Europäisches Patentamt

(19) European Patent Office

Office européen des brevets

(11) Numéro de publication : **0 166 659**
**B1**

(12) **FASCICULE DE BREVET EUROPÉEN**

(45) Date de publication du fascicule du brevet :
30.08.89

(21) Numéro de dépôt : 85401236.6

(22) Date de dépôt : 20.06.85

(51) Int. Cl.⁴ : **C 12 N 15/00**, C 12 N 1/16,
C 12 P 13/08 // (C12N1/16,
C12R1:645)

(54) Vecteurs de transformation de la levure yarrowia lipolytica, procédé de transformation et levure transformée.

(30) Priorité : 26.06.84 FR 8410030
02.04.85 FR 8504990

(43) Date de publication de la demande :
02.01.86 Bulletin 86/01

(45) Mention de la délivrance du brevet :
30.08.89 Bulletin 89/35

(84) Etats contractants désignés :
AT BE CH DE FR GB IT LI LU NL SE

(56) Documents cités :
EP—A— 0 138 508
BIOLOGICAL ABSTRACTS, vol. 77, 1984, page 2095,
no. 19134, Philadelphia, US; H. EIBEL et al.: "Identification of the cloned Saccharomyces cerevisiae LYS2
gene by an integrative transformation approach", &
MOL. GEN. GENET. 191(1): 66-73. 1983
CHEMICAL ABSTRACTS, vol. 99, no. 9, 29 août 1983,
page 340, no. 67279k, Columbus, Ohio, US; D.P.
GROVES et al.: "Isolation and characterization of a
double-stranded RNA virus-like particle from the
yeast Yarrowia lipolytica", & CURR. GENET. 1983,
7(3), 185-190
CURRENT TOPICS IN MICROBIOLOGY AND IMMU-
NOLOGY, vol. 96, 1982, pages 101-117, US; A. HINNEN
et al.: "Vectors for cloning in yeast"

(73) Titulaire : INSTITUT NATIONAL DE LA RECHERCHE
AGRONOMIQUE (INRA)
145, rue de l'Université
F-75341 Paris Cédéx 07 (FR)

(72) Inventeur : Gaillardin, Claude
24, rue d'Hautpoul
F-75019 Paris (FR)
Inventeur : Heslot, Henri
29, rue Rousselet
F-75007 Paris (FR)
Inventeur : Ribet, Anne-Marie
35, rue Faidherbe
F-75011 Paris (FR)

(74) Mandataire : Warcoin, Jacques et al
Cabinet Régimbeau 26, avenue Kléber
F-75116 Paris (FR)

Description

La présente invention concerne la transformation de levures Yarrowia, en particulier de Y. lipolytica, par des vecteurs de clonage.

On connaît actuellement des techniques permettant de transformer un certain nombre de· levures telles que Saccharomyces cerevisiae, Schizosaccharomyces pombe et Kluyveromyces lactis ; mais, jusqu'à maintenant, il n'a pas été possible d'obtenir la transformation de Yarrowia par des vecteurs plasmidiques.

Or, la levure Yarrowia est utilisée actuellement dans la préparation de différents acides, notamment l'acide citrique, et présente la particularité d'être capable de préparer des protéines exocellulaires, ce qui la différencie de Saccharomyces cerevisiae et Kluyveromyces lactis, et présente évidemment, sur le plan industriel, un avantage considérable puisqu'il n'est pas nécessaire d'éclater les cellules afin de récupérer les protéines qui ont pu être synthétisées.

Le problème essentiel rencontré lors de la mise au point d'un système de transformation pour Yarrowia lipolytica est le choix d'un marqueur de sélection et d'une souche receveuse.

Les souches de Yarrowia sont en général résistantes aux antibiotiques de type gentamycine et en particulier à l'antibiotique G418 couramment utilisé pour sélectionner les levures, et, en particulier, il a été observé que la traduction de messagers par des ribosomes de Y. lipolytica n'est que très peu sensible à cet antibiotique, ce qui exclut de pouvoir sélectionner des transformants en utilisant des vecteurs portant le transposon Tn609 codant pour la résistance à cet antibiotique.

De même, les systèmes classiques utilisés dans le cadre de la transformation de S. cerevisiae, à savoir l'utilisation du gène URA3+, ont pu également être utilisés dans la transformation de Schizosaccharomyces pombe et de Kluyveromyces lactis. Toutefois, l'utilisation de plasmides portant ce gène marqueur implique de disposer de mutants ura3-, ce qui est difficilement réalisable dans le cadre de Y. lipolytica car ces mutants sont trop labiles et reversent trop facilement.

Dans le cadre de la présente invention, on a pu mettre en évidence des mutants stables, auxotrophes, pour la lysine et mettre en évidence la possibilité de complémenter cette auxotrophie par l'intermédiaire d'un plasmide vecteur portant le gène LYS2+ de S. cerevisiae.

Le gène LYS2+ de S. cerevisiae a été cloné dans pBR322 (voir Eibel H. et coll. 1983). Ce plasmide pBR322 portant le gène LYS2+ de S. cerevisiae sera appelé ci-après plasmide Ylp333. Ce gène code pour l'α-aminoadipate réductase et doit correspondre au locus complexe LYS2-LYS-3 de Y. lipolytica.

C'est pourquoi la présente invention concerne des vecteurs de clonage dans une souche de Yarrowia, en particulier dans Y. lipolytica, caractérisés en ce que ces vecteurs comportent au moins un fragment d'ADN de Yarrowia, en particulier Y. lipolytica, ainsi que le gène LYS2+ de S. cerevisiae.

De préférence, ce vecteur de clonage comportera tout ou partie de l'ADN plasmidique d'un vecteur bactérien tel que pBR322.

En outre, ce vecteur pourra comporter, afin d'exprimer une protéine particulière, outre le gène codant pour ladite protéine, les différents éléments permettant d'en assurer l'expression dans Y. lipolytica, en particulier une séquence assurant la promotion dudit gène à exprimer.

Pour la mise en œuvre de ce vecteur, on transformera une souche de Y. lipolytica lys2- et on sélectionnera les transformants devenus lys2+.

L'étude de l'ADN transformant montre qu'aucun plasmide libre n'est détecté, ni par hybridation de l'ADN total non restreint ni par retransformation d'E. coli. L'ADN transformant est intégré dans le génome à raison d'une à au moins 10 copies par génome suivant les transformants. La structure de ces insertions multiples n'est pas modifiée après 30 générations en milieu sélectif. Après restriction par l'enzyme PstI et religation de l'ADN des souches transformées, on peut réisoler dans E. coli des fragments de Ylp333 associés à des séquences uniques de Y. lipolytica.

En outre, le gène de résistance à la tétracycline d'E. coli est maintenu sans modification dans la levure et s'exprime normalement dans E. coli après réextraction de Y. lipolytica.

Enfin, les souches ainsi transformées sont hyperproductrices de lysine, en particulier lorsque plusieurs copies du gène LYS2+ ont été intégrées dans leur génome.

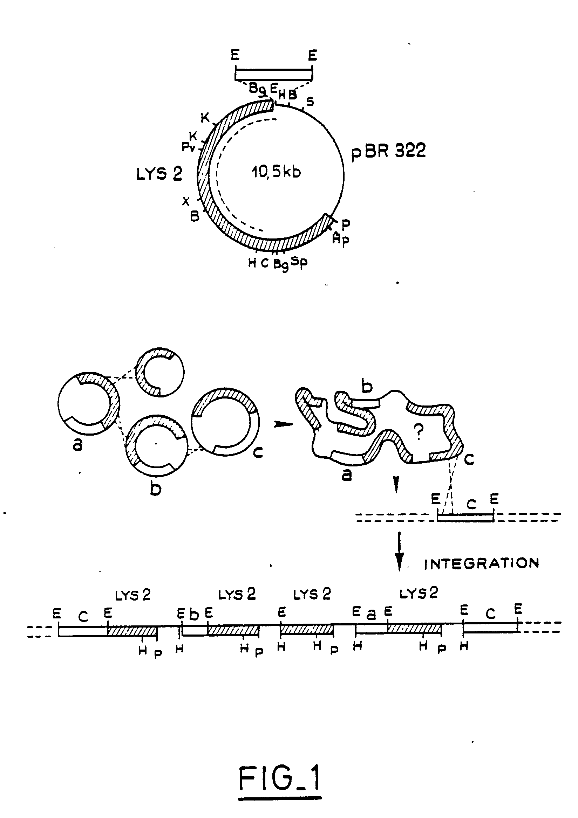

Les recherches récentes suggèrent que ces intégrations multiples résultent de la pénétration de plusieurs plasmides dans certaines cellules (cotransformation). Selon toute vraisemblance, ces plasmides se recombinent entre leurs régions homologues (LYS2+ ou pBR322) en formant des cointégrats. Ces molécules polymériques s'intègrent ensuite dans le chromosome par échange entre régions homologues (ADN de Y. lipolytica du polymère et ADN du chromosome). Les copies multiples de LYS2+ sont localisées côte à côte dans un chromosome déterminé qui varie selon le transformant, comme cela est explicité dans la figure 1.

Sur la figure 1, la banque d'ADN de Y. lipolytica est intégrée dans un plasmide pBR322 portant LYS2 (schématisé en A). Après transformation, ces plasmides se recombinent entre eux par l'intermédiaire de leurs régions homologues pour former des cointégrats (polymère) représentés en B. Ce polymère s'intègre alors dans un chromosome grâce aux séquences homologues, ce qui conduit à des copies multiples de LYS2 intégrées.

On a montré récemment que ce système de transformation permet l'intégration simultanée de

2

plusieurs plasmides distincts indépendants, si les cellules à transformer sont mises en présence d'un mélange. D'une façon plus précise, ce mécanisme permet l'intégration efficace de n'importe quel ADN circulaire présentant une homologie avec le plasmide qui porte le marqueur sélectionné (on a utilisé LYS2$^+$, mais on pourrait en prendre un autre).

C'est pourquoi la présente invention concerne également une combinaison de vecteurs destinée à l'intégration d'une séquence d'ADN dans le génome de Yarrowia, par exemple Y. lipolytica qui comprend :

a) un plasmide 1 comportant au moins ladite séquence d'ADN à intégrer et une région homologue du plasmide 2 ;

b) un plasmide 2, dit « d'intégration », comportant au moins une région homologue du génome de Y. lipolytica, et le gène marqueur LYS2$^+$ de S. cerevisiae s'exprimant dans Y. lipolytica.

Afin de faciliter la mise en œuvre, la séquence homologue entre le plasmide 1 et le plasmide 2 sera une séquence de pBR322 couramment utilisée pour le clonage de gènes hétérologues.

Ce plasmide 2 d'intégration ainsi conçu peut être utilisé pour intégrer n'importe quel gène, ou séquence d'ADN, cloné dans pBR322 qui constituera le plasmide 1.

De façon générale, le plasmide d'intégration sera incapable de se répliquer de façon autonome dans Y. lipolytica.

A titre d'exemple, on peut intégrer dans le génome de Y. lipolytica les gènes suivants clonés sur un plasmide 1 :

SUC2 codant pour l'invertase de S. cerevisiae,

PHO5 codant pour la phosphatase alcaline de S. cerevisiae,

lacZ codant pour la β-galactosidase de E. coli, le gène codant pour une α-amylase thermostable de B. licheniformis.

La présente addition concerne également un procédé d'intégration d'une séquence d'ADN déterminée dans Y. lipolytica, caractérisé en ce qu'on effectue la cotransformation d'une souche de Y. lipolytica par au moins une combinaison de vecteurs telle que décrite précédemment.

La souche transformée sera, de préférence, une souche auxotrophe, le plasmide portant un gène marqueur complémentant cette auxotrophie, par exemple le gène LEU2$^+$ qui complémentera une souche leu$^-$.

D'autres caractéristiques et avantages de la présente addition apparaîtront dans l'exemple ci-après décrit en se référant aux dessins annexés sur lesquels :

la figure 1 représente un schéma de l'intégration du gène LYS2 sous forme de copies multiples,

la figure 2 représente la structure de pRB58,

la figure 3 représente le schéma de la recombinaison entre pRB58 et B6,

la figure 4 montre l'hybridation de l'ADN extrait d'un clone de Y. lipolytica transformé par B6 et pRB58.

Exemple 1

Mise en évidence de la complémentation de l'auxotrophie lys2$^-$ par le gène LYS2$^+$

Afin de mettre en évidence que le gène LYS2$^+$ pouvait complémenter une mutation lys2$^-$ de Y. lipolytica, on a effectué les fusions de protoplastes suivants :

— la fusion lys2$^+$ S. cerevisiae × lys2$^-$ Y. lipolytica

— la fusion lys2$^-$ S. cerevisiae × lys2$^-$ Y. lipolytica

— la fusion lys2$^+$/B6-61 S. cerevisiae × lys2$^-$ Y. lipolytica.

La première fusion est lys2$^+$ alors que la seconde fusion est lys2$^-$, ceci démontre que la complémentation est possible. La troisième fusion correspond à une souche de S. cerevisiae lys2$^-$ transformée par le plasmide B6-61 qui est un dérivé de YIp333 portant un fragment EcoRI de Y. lipolytica qui permet sa réplication dans S. cerevisiae. Le fait que la fusion soit lys$^+$ a montré qu'il était envisageable de transformer Y. lipolytica directement avec un plasmide analogue à B6-61.

Exemple 2

Construction de plasmides vecteurs pour Y. lipolytica

Le plasmide de base utilisé pour la réalisation des plasmides selon la présente invention est le plasmide YIp333 décrit dans l'article de Eibel H. et coll., 1983, et qui comporte essentiellement le gène LYS2 de S. cerevisiae cloné dans le plasmide bactérien pBR322.

3

Les expériences conduites par Philippsen ont mis en évidence que ce plasmide était capable de complémenter la mutation lys2 dans S. cerevisiae.

Ce plasmide Ylp333 digéré par EcoRI est ligué avec un « shotgun » d'ADN total de Y. lipolytica traité par EcoRI (banque B6, 18 000 hybrides, taille moyenne de l'insert : 6,2 kb).

On obtient de cette façon un grand nombre de plasmides selon la présente invention, c'est-à-dire comportant le gène LYS2+ et des séquences aléatoires prélevées sur Y. lipolytica.

## Exemple 3

### Transformation de Y. lipolytica

On utilise pour cette transformation une souche de Y. lipolytica 8601-1 (LYS1-5 lys2, adel).

LYS1-5 supprime le rétro-contrôle par la lysine du premier enzyme de la voie de biosynthèse de ladite lysine.

La transformation de la souche de Y. lipolytica par les vecteurs plasmidiques construits précédemment est effectuée selon la technique décrite pour S. cerevisiae dans l'ouvrage Methods in Yeast Genetic, 1981.

Les protoplastes ont été mis à régénérer sur un milieu minimum supplémenté en adénine.

Après 4 jours d'incubation, on a observé, à saturation d'ADN (5 μg/ml), environ 1 colonie LYS+ pour $5.10^5$ protoplastes régénérés, sur un fond de microcolonies abortives (environ 1 % des protoplastes viables ont été transformés mais ne maintiennent pas l'ADN transformant).

Toutes les colonies LYS+ s'hybrident à une sonde Ylp333, alors que les colonies lys⁻ n'hybrident plus. Ceci permet de les distinguer d'éventuels revertants.

La stabilité des transformants a été étudiée et mesurée après 10 générations en milieu sélectif.

Les résultats observés sont rappelés dans le tableau.

### Etude de l'ADN Transformant

Aucun plasmide libre n'a été détecté, ni par hybridation de l'ADN total non restreint ni par retransformation d'E. coli. Après restriction de l'ADN total de ces transformants par différents enzymes de restriction, on peut conclure que :

— les transformants dénommés 621 et 622 ne portent qu'une copie (remaniée) du vecteur ;

— les transformants 641, 642 et 643 portent 2 à 3 copies du vecteur, celui-ci n'est pas remanié (après restriction par EcoRI on sépare le fragment Ylp333 de l'ADN total, une restriction par PstI donne 2 bandes par copie intégrée) ;

— les transformants 644 et 651 portent 10 copies ou plus du vecteur non remanié.

## Exemple 4

### Effet du nombre de copies LYS2+ sur le pool en lysine libre

La souche 8601-1 est dérégulée pour le premier enzyme de la voie de biosynthèse de la lysine.

On sait que dans une souche sauvage portant cette mutation le flux des intermédiaires est ralenti au niveau de l'aminoadipate réductase (locus LYS2) et qu'on ne déréprime pas cette activité dans les mutants affectés dans le contrôle général, on pouvait donc penser qu'une augmentation du pool de lysine libre devait résulter d'une augmentation du nombre de copies du gène LYS2+.

Cette hypothèse a été testée avec le gène cloné LYS2+ de S. cerevisiae présent sur Ylp333. On a pu constater :

— que le « pool » observé après insertion d'une seule copie de LYS2 est supérieur à celui d'une souche témoin de Y. lipolytica LYS2+ ;

— que l'augmentation du nombre de copies de LYS2 conduit à une augmentation du pool de lysine libre.

Les résultats mesurés sont rappelés dans le tableau ci-après :

### Tableau

| SOUCHE | STABILITE (%) | NOMBRE DE COPIES | | nMoles lys/mg poids sec |
|---|---|---|---|---|
| | | LYS2 Y.lipo. | LYS2 S.cerev. | |
| Réf. | | 1 | 0 | 100 |
| 621 | 99,9 | 0 | 1 | 219 |

## EP 0 166 659 B1

### Tableau (Suite)

| SOUCHE | STABILITE (%) | NOMBRE DE COPIES | | nMoles lys/mg poids sec |
|--------|---------------|------------------|------|------------|
| | | LYS2 Y.lipo. | LYS2 S.cerev. | |
| 622 | 99,9 | O | 1 | 214 |
| 641 | 33,- | O | 2-3 | 565 |
| 642 | 77,- | O | 2-3 | 483 |
| 643 | 26,- | O | 2-3 | 567 |
| 644 | 19 | O | 10 | 880 |
| 651 | 91 | O | 10 | 800 |

(Toutes les cultures sont faites en milieu minimum + ade). (La stabilité est estimée après 10 générations sur milieu complet).

Ces expériences mettent en évidence la possibilité d'une amplification génique correspondant dans ce cas particulier à une étape limitante de la biosynthèse de la lysine.

Les souches ainsi obtenues sont devenues hyperproductrices de lysine.

### Exemple 5

Les techniques mises en œuvre sont les mêmes que celles décrites précédemment.

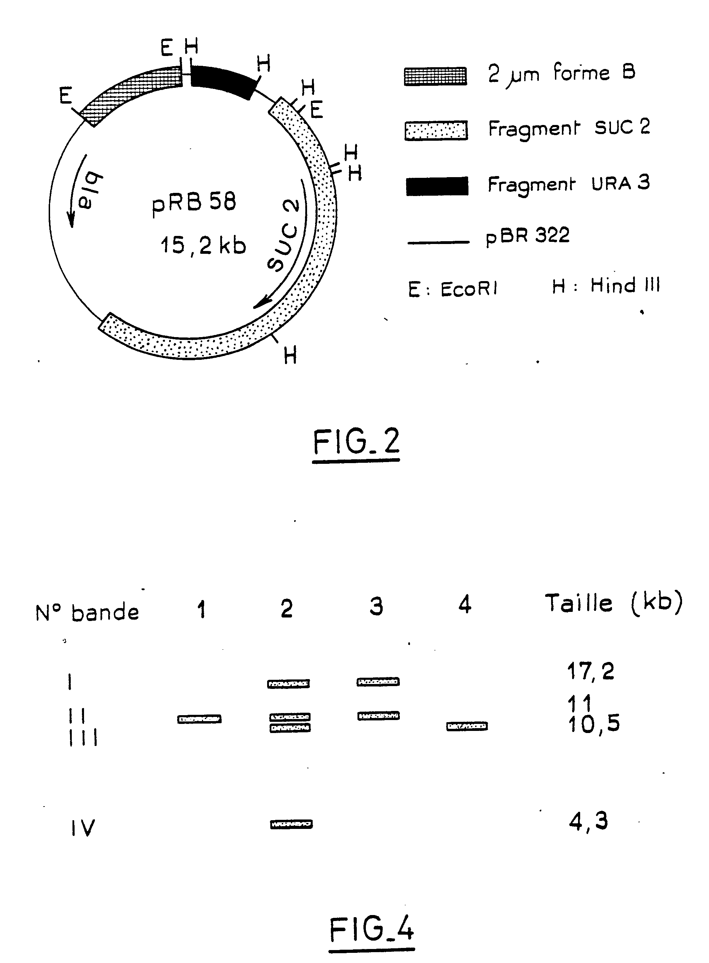

Le plasmide pRB58 représenté sur la figure 2 est un plasmide dérivé de pBR322 portant au site EcoRI de pBR322 une insertion de 2,2 kb comprenant un fragment du plasmide 2 μm de S. cerevisiae (forme B) portant l'origine de réplication du plasmide 2μm ; une insertion de 1,1 kb au site HindIII de pBR322 comprenant un fragment d'ADN chromosomique de S. cerevisiae correspondant au gène URA3 ; une insertion de 8,5 kb d'ADN chromosomique de S. cerevisiae au site BamHI de pBR322, ce dernier fragment porte le gène SUC2 codant pour l'invertase (Carlson et Botstein (1983) Cell.28, p. 145-154).

Il existe donc une homologie de séquence entre les parties pBR322 de B6 et de pRB58 permettant la formation éventuelle de concatémères portant 4 types de séquences recombinées.

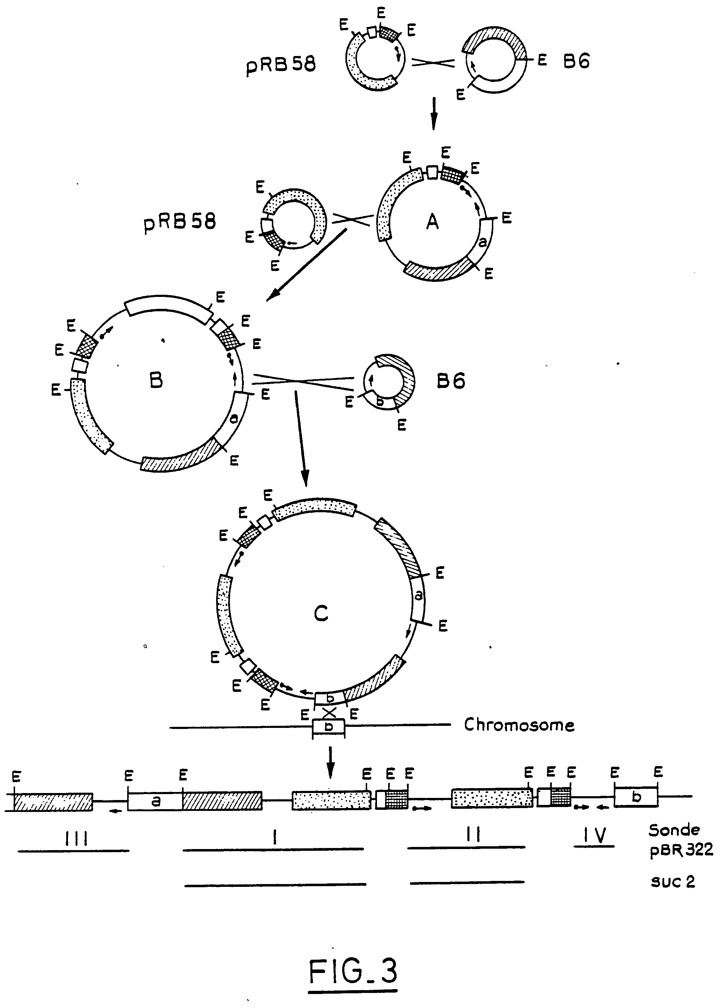

Comme cela est représenté sur la figure 3, les plasmides pRB58 et B6 peuvent se recombiner par leurs séquences homologues de pBR322 pour donner un plasmide intermédiaire A. Ce dernier plasmide A peut se recombiner avec pRB58 pour donner le plasmide B qui peut lui-même se recombiner avec le plasmide B6 pour donner le plasmide C (bien entendu, ces recombinaisons ne sont données qu'à titre d'exemple). Ce plasmide C peut s'intégrer dans le chromosome par la séquence homologue notée b. Ceci conduit à une structure chromosomique linéaire qui est schématisée en bas de la figure 4. Une restriction EcoRI et une hybridation avec pBR322 doit conduire à des fragments notés de I à IV.

Après transformation de la souche de Y. lipolytica 8601.1 par un mélange de B6 et de pRB58, 12 clones LYS+ ont été isolés et leur ADN a été extrait.

Cet ADN a été digéré par l'enzyme EcoRI et les fragments ont été séparés sur gel d'agarose puis transférés sur un filtre de nitrocellulose. Celui-ci a été hybridé avec une sonde pBR322 (bande 2) et, dans une seconde expérience, avec une sonde spécifique de SUC2 (bande 3) (fragment HindIII de 3 kb de l'insertion de 8,5 kb). (La bande 1 correspond à pRB58 coupé par EcoRI hybridé avec pBR322 et la bande 4 à Ylp333 coupé par EcoRI hybridé avec pBR322).

Comme le montre la figure 4, on observe, dans des clones analysés, l'ADN SUC2 intégré sous forme de copies multiples. Le même profil de restriction a été observé dans les 12 clones cotransformés, l'intensité relative des différentes bandes I, II, III, IV étant variable et reflétant le nombre de copies de pRB58 et de Ylp333 qui ont participé à la formation du polymère.

La même expérience a été reprise en cotransformant B6 avec différents plasmides dérivés de pBR322 portant des gènes d'origine diverse : PHO5 (phosphatase de S. cerevisiae), amy (α-amylase de B. licheniformis), lacZ (β-galactosidase d'E. coli). Dans tous les cas, les transformants LYS+ isolés contenaient également l'ADN cotransformé.

### Références

EIBEL H. et P. PHILIPPSEN (1983) Molec. Gen. Genet. 191, 66-73.

Methods in Yest Genetics, p. 91-92, Cold Spring Harbor Laboratory (ed), Cold Spring Harbor N.Y. (1981).

**Revendications**

5

1. Vecteur de clonage dans une souche de Yarrowia, caractérisé en ce qu'il comporte au moins un fragment d'ADN de Yarrowia destiné à assurer l'intégration du vecteur dans le genome de ladite souche, ainsi que le gène LYS2$^+$ de S. cerevisiae.

2. Vecteur selon la revendication 1, caractérisé en ce qu'il comporte, en outre, un fragment de plasmide bactérien.

3. Vecteur selon la revendication 2, caractérisé en ce que le plasmide bactérien est pBR322.

4. Vecteur selon l'une des revendications 1 à 3, caractérisé en ce que le fragment d'ADN de Yarrowia est un fragment EcoRI.

5. Combinaison de vecteurs destinée à l'intégration d'une séquence d'ADN déterminée dans le génome de Yarrowia lipolytica qui comprend :

   a) un plasmide 1 comportant au moins ladite séquence d'ADN déterminée à intégrer et une région homologue du plasmide 2, et

   b) un plasmide 2 dit d'intégration comportant au moins une région homologue du génome de Y. lipolytica, et le gène marqueur LYS2$^+$ de S. cerevisiae s'exprimant dans Y. lipolytica.

6. Combinaison selon la revendication 6, caractérisée en ce que le plasmide d'intégration est incapable de se répliquer chez Y. lipolytica.

7. Combinaison selon l'une des revendications 6 et 7, caractérisée en ce que la région homologue du plasmide d'intégration est une région de pBR322.

8. Combinaison selon l'une des revendications 6 à 8, caractérisée en ce que le plasmide 1 est un plasmide pBR322 portant le gène SUC2 codant pour l'invertase de S. cerevisiae.

9. Combinaison selon l'une des revendications 6 à 8, caractérisée en ce que le plasmide 1 est un plasmide pBR322 portant le gène PHO5 codant pour la phosphatase alcaline de S. cerevisiae.

10. Combinaison selon l'une des revendications 6 à 8, caractérisée en ce que le plasmide 1 est un plasmide pBR322 portant le gène lacZ codant pour la β-galactosidase de E. coli.

11. Combinaison selon l'une des revendications 6 à 8, caractérisée en ce que le plasmide 1 est un plasmide pBR322 portant le gène codant pour une α-amylase thermostable de B. licheniformis.

12. Procédé d'intégration d'une séquence d'ADN déterminée dans Y. lipolytica, caractérisé en ce qu'on effectue la cotransformation d'une souche de Y. lipolytica par au moins une combinaison de vecteurs selon l'une des revendications 6 à 12.

13. Levure Y. lipolytica ayant une séquence d'ADN déterminée intégrée dans son génome obtenue par la mise en œuvre du procédé selon la revendication 13.

14. Souche de Yarrowia transformée par un vecteur selon l'une des revendications 1 à 5.

15. Souche selon la revendication 15, caractérisée en ce qu'il s'agit d'une souche de Y. lipolytica.

16. Souche selon l'une des revendications 15 et 16, caractérisée en ce qu'elle comporte plusieurs copies du gène LYS2+ intégrées dans son génome.

17. Procédé de préparation de la lysine, caractérisé en ce qu'on fait fermenter sur un milieu de culture approprié une souche selon l'une des revendications 15 à 17.

## Claims

1. A cloning vector in a culture of Yarrowia characterised in that it comprises at least a fragment of DNA of Yarrowia, intended to ensure insertion of the vector in the genome of said culture, as well as the gene LYS2$^+$ of S. cerevisiae.

2. A vector according to claim 1 characterised in that it comprises, in addition, a fragment of bacterial plasmid.

3. A vector according to claim 2 characterised in that the bacterial plasmid is pBR322.

4. A vector according to one of claims 1 to 3 characterised in that the fragment of DNA of Yarrowia is an EcoRI fragment.

5. A combination of vectors, intended to insert a predetermined sequence of DNA into the genome of Yarrowia lipolytica which comprises :

   a) a plasmid 1 comprising at least the said predetermined sequence of DNA to be inserted and a sequence complimentary to plasmid 2, and

   b) an insertion plasmid 2 comprising at least a sequence complimentary to the genome of Y. lipolytica, and the marker gene LYS2$^+$ of S. cerevisiae expressed in Y. lipolytica.

6. A combination according to claim 5 characterised in that the insertion plasmid is incapable of self replication in Y. lipolytica.

7. A combination according to one of claims 5 or 6 characterised in that the sequence complimentary to the insertion plasmid is a sequence of pBR322.

8. A combination according to one of claims 5 to 7 characterised in that the plasmid 1 is a plasmid pBR322 carrying the genetic coding SUC2 of S. cerevisiae for sucrase.

9. A combination according to one of claims 5 to 7 characterised in that plasmid 1 is a plasmid pBR322 carrying the genetic coding PHO5 of S. cerevisiae for alkaline phosphatase.

10. A combination according to one of claims 5 to 7 characterised in that the plasmid 1 is a plasmid pBR322 carrying the genetic coding lacZ of E. Coli for β-galactosidase.

6

11. A combination according to one of claims 5 to 7 characterised in that the plasmid 1 is a plasmid pBR322 carrying the genetic coding of B. licheniformis for a thermostable α-amylase.

12. A method of inserting a predetermined sequence of DNA in Y. lipolytica characterised in that one effects the co-transformation of a culture of Y. lipolytica by means of a combination of vectors according to one of claims 6 to 11.

13. Yeast Y. lipolytica having a predetermined sequence of DNA inserted in its genome, obtained by utilisation of the process in accordance with claim 12.

14. A culture of Yarrowia transformed by a vector according to one of claims 1 to 4.

15. A culture according to claim 14 characterised in that the culture concerned is Y. lipolytica.

16. A culture according to one of claims 14 and 15 characterised in that it includes several copies of the gene LYS2$^+$ inserted into its genome.

17. A method of preparation of lysine characterised in that a culture according to one of claims 14 to 16 is fermented in an appropriate culture medium.


**Patentansprüche**

1. Klonierungsvektor in einem Yarrowia-Stamm, dadurch gekennzeichnet, daß er mindestens ein den Vektoreinbau in das Genom dieses Stamms gewährleistendes Yarrowia-DNA-Fragment sowie das Gen LYS2$^+$ von S. cerevisiae enthält.

2. Vektor nach Anspruch 1, dadurch gekennzeichnet, daß er außerdem ein bakterielles Plasmidfragment enthält.

3. Vektor nach Anspruch 2, dadurch gekennzeichnet, daß das bakterielle Plasmid pBR322 ist.

4. Vektor nach einem der Ansprüche 1 bis 3, dadurch gekennzeichnet, daß das Yarrowia-DNA-Fragment ein EcoRI-Fragment ist.

5. Vektorenkombination, die zum Einbau einer bestimmten DNA-Sequenz in das Genom von Yarrowia lipolytica dient, umfassend :

a) ein Plasmid 1, umfassend mindestens die zum Einbau bestimmte DNA-Sequenz und einen homologen Bereich zu einem Plasmid 2 und

b) ein Einbauplasmid 2 mit mindestens einem homologen Bereich des Y. lipolytica-Genoms und dem sich in Y. lipolytica exprimierenden Markergen LYS2$^+$ von S. cerevisiae.

6. Kombination nach Anspruch 5, dadurch gekennzeichnet, daß das Einbauplasmid unfähig ist, sich in Y. lipolytica zu replizieren.

7. Kombination nach einem der Ansprüche 5 und 6, dadurch gekennzeichnet, daß der homologe Bereich vom Einbauplasmid ein Bereich von pBR322 ist.

8. Kombination nach einem der Ansprüche 5 bis 7, dadurch gekennzeichnet, daß das Plasmid 1 aus einem das Gen SUC2 mit Kodierung für die Invertase von S. cerevisiae tragenden Plasmid pBR322 besteht.

9. Kombination nach einem der Ansprüche 5 bis 7, dadurch gekennzeichnet, daß das Plasmid 1 aus einem das Gen PHO5 mit Kodierung für die alkalische Phosphotase von S. cerevisiae tragenden Plasmid pBR322 besteht.

10. Kombination nach einem der Ansprüche 5 bis 7, dadurch gekennzeichnet, daß das Plasmid 1 aus einem das Gen lacZ mit Kodierung für die β-Galactosidase von E. coli tragenden Plasmid pBR322 besteht.

11. Kombination nach einem der Ansprüche 5 bis 7, dadurch gekennzeichnet, daß das Plasmid 1 aus einem das Gen mit Kodierung für eine thermostabile α-Amylase von B. licheniformis tragenden Plasmid pBR322 besteht.

12. Verfahren zum Einbau einer bestimmten DNA-Sequenz in Y. lipolytica, dadurch gekennzeichnet, daß man die Cotransformation eines Y. lipolytica-Stammes mit mindestens einer Kombination von Vektoren nach einem der Ansprüche 5 bis 11 durchführt.

13. Y. lipolytica-Hefe mit einer bestimmten, in sein Genom eingebauten DNA-Sequenz, erhalten bei der Durchführung des Verfahrens nach Anspruch 12.

14. Mit einem Vektor nach einem der Ansprüche 1 bis 4 transformierter Yarrowia-Stamm.

15. Stamm nach Anspruch 14, dadurch gekennzeichnet, daß es sich um einen Y. lipolytica-Stamm handelt.

16. Stamm nach einem der Ansprüche 14 und 15, dadurch gekennzeichnet, daß er in sein Genom mehrere Kopien des Gens LYS2$^+$ eingebaut enthält.

17. Verfahren zur Herstellung von Lysin, dadurch gekennzeichnet, daß man in einem geeigneten Kulturmedium einen Stamm nach einem der Ansprüche 14 bis 16 wachsen läßt.

FIG_1

## FIG. 2

| N° bande | 1 | 2 | 3 | 4 | Taille (kb) |
|----------|---|---|---|---|-------------|
| I | | ▭ | ▭ | | 17,2 |
| II | ▭ | ▭ | ▭ | | 11 |
| III | | ▭ | ▭ | ▭ | 10,5 |
| IV | | ▭ | | | 4,3 |

Legend:
2 μm forme B
Fragment SUC 2
Fragment URA 3
pBR 322
E : EcoRI    H : Hind III

## FIG. 4

pRB 58   B6

pRB 58   A

B   B6

C

Chromosome

III        I        II        IV        Sonde pBR 322

suc 2

<u>FIG_3</u>